(19) Europäisches Patentamt
European Patent Office
Office européen des brevets

(11) Publication number: **0 132 987**
**B1**

(12) **EUROPEAN PATENT SPECIFICATION**

(45) Date of publication of patent specification: **16.09.87**

(21) Application number: **84304855.4**

(22) Date of filing: **17.07.84**

(51) Int. Cl.[4]: **C 07 D 501/04,**
C 07 D 498/04,
C 07 D 499/08,
C 07 D 487/04, C 07 D 471/04
// (C07D498/04, 265:00, 205:00),(C07D487/04, 209:00, 205:00),(C07D471/04, 221:00, 205:00)

(54) **Improvements in or relating to deesterification to acids.**

(30) Priority: **22.07.83 US 516219**

(43) Date of publication of application:
**13.02.85 Bulletin 85/07**

(45) Publication of the grant of the patent:
**16.09.87 Bulletin 87/38**

(84) Designated Contracting States:
**BE CH DE FR GB IT LI LU NL SE**

(56) References cited:
**GB-A-1 569 040**
**GB-A-1 582 960**

(73) Proprietor: **ELI LILLY AND COMPANY**
**307, East McCarty Street**
**Indianapolis Indiana 46285 (US)**

(72) Inventor: **Fisher, Jack Wayne**
**2533 Fox Harbour Drive**
**Indianapolis Indiana 46227 (US)**

(74) Representative: **Crowther, Terence Roger et al**
**Erl Wood Manor**
**Windlesham Surrey GU20 6PH (GB)**

**EP 0 132 987 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

Compounds such as the penicillin and cephalosporin antibiotics and their nuclear analogs are complex organic molecules which generally possess one or more functional groups. The synthesis of such compounds routinely requires the use of protecting groups for the functional groups that would enter into, if unprotected, unwanted side reactions leading to undesired products. The most common functional group present in compounds such as penicillins and cephalosporins is the carboxylic acid group. The typical method of protecting the carboxylic acid group during reactions is to convert it to an ester. Once the desired reaction has taken place elsewhere in the molecule, the ester must be converted back to the carboxylic acid group, which generally is required for biological activity. Because compounds such as the penicillins and cephalosporins and their nuclear analogs are very sensitive to normal de-esterification conditions such as strong acid or strong base, methods for de-esterification must be very mild.

A process for deprotecting a carboxy group in the presence of a Lewis acid is disclosed in GB—A—1 569 040.

In accordance with the invention, there is provided a de-esterification process for esters of organic carboxylic acids that is unusually mild and efficient.

In particular, the invention provides a process for preparing a compound of Formula (I):

$$R^1—COOH \qquad (I)$$

which comprises reacting a lower alkyl thiol and a Lewis acid with an ester of Formula (II):

$$R^1—COOR^2 \qquad (II)$$

in which $R^1$ is the residue of a penicillin cephalosporin or a nuclear analogue thereof or of an amino acid and $R^2$ is an organic ester forming group capable of forming a stabilized carbonium ion.

In a preferred embodiment, $R^2$ is diphenylmethyl or 2,4,6-trimethylbenzyl. In another preferred embodiment, $R^1$ is a penicillin, cephalosporin, carbapenicillin, carbacephalosporin, or oxacephalosporin nucleus moiety.

The process provided by this invention is carried out by reacting an ester of a carboxylic acid with a lower alkyl thiol and a Lewis acid. By "lower alkyl thiol" is meant a compound such as methanethiol, ethanethiol, *n*-propanethiol, *n*-butanethiol and *iso*-hexanethiol. A preferred lower alkyl thiol is ethanethiol. The term "Lewis acid" carries its art recognized meaning of a substance that can take up an electron pair to form a covalent bond. Boron trifluoroide is a widely recognized Lewis acid, and is a preferred Lewis acid of this invention. It generally is employed as the commercially available diethyl etherate complex. Other Lewis acids which may be used in the present process include stannic chloride, zinc chloride and the like.

The de-esterification process of this invention generally is performed by combining about equimolar quantities of an ester of a carboxylic acid, a Lewis acid and a lower alkyl thiol. If desired, a small excess, for instance about 0.01 to about 1.0 molar excess, of the Lewis acid and/or the lower alkyl thiol may be employed to ensure complete de-esterification of the ester. Further, if desired, the reaction may be carried out in an unreactive organic solvent. Solvents routinely employed include halogenated hydrocarbons such as chloroform, dichloromethane, 1,2-dichloroethane; aromatic solvents such as benzene, toluene, or xylene; and ketones such as acetone or methyl ethyl ketone. A preferred reaction solvent is a halogenated hydrocarbon such as dichloromethane.

The process of this invention generally is complete after about one-half to about eight hours when carried out at a temperature of about −20 to about +50°C. In a preferred embodiment, the process is conducted at about 20 to about 30°C. Once the de-esterification reaction is substantially complete, the carboxylic acid product readily can be isolated, if desired, by routine methods, for example by simply filtering the product or removing the reaction solvent. The product can be further purified, if needed, by standard extraction techniques, chromatography, crystallization, salt formation or other known purification techniques.

The process of this invention is quite general and may be performed on any carboxylic acid ester in which the ester forming group is capable of forming a stabilized carbonium ion. Carbonium ions are well-known intermediates in several kinds of organic reactions. Carbonium ions are organic species that carry a positive charge on a central carbon atom, and usually are very short lived. A stabilized carbonium ion is one that possesses a resonance or hyperconjugation energy greater than about 84 kcal/mole, the resonance energy for the *tert*-butyl carbonium ion. Typical ester forming groups which form stabilized carbonium ions may be employed in the substrates for the present process include bulky alkyl groups such as *tert*-butyl and diphenylmethyl; alkylated benzyl groups such as 2,4,6-trimethylbenzyl and pentamethylbenzyl; alkoxybenzyl groups such as *p*-methoxybenzyl, and polycyclic methyl groups such as 9-anthrylmethyl.

As noted above, the present de-esterification process is quite general and can be carried out on esters in which $R^1$ in the above formula is basically any organic group. The mildness of the process makes it particularly applicable to sensitive compounds such as penicillins and cephalosporins and their nuclear analogs. In a preferred embodiment, $R^1$ in the above formula is a penicillin moiety of Formula (III):

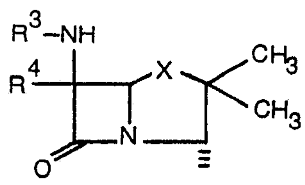

(III)

or a cephalosporin moiety of Formula (IV):

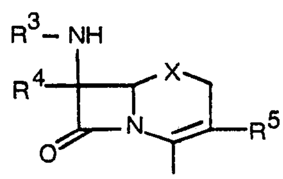

(IV)

in which $R^3$ is an acyl residue, $R^4$ is hydrogen, alkoxy or alkylthio, X is S, O, or $CH_2$, and $R^5$ is hydrogen, halogen, or an organic group commonly found in cephalosporin compounds. Typical $R^5$ groups include methyl, chloro and acetoxymethyl, however, a wide variety of other pharmaceutically-acceptable groups are known and may be employed.

The nature of the group defined in the above formula by $R^1$ is not critical to the process of this invention because the process operates only on the carboxylic acid ester moiety. If a carboxylic acid ester group such as a diphenylmethyl ester or the like is located at another site in the molecule, for instance as part of the $R^3$ group, some de-esterification at that ester group will occur also. If desired, any carboxylic acid groups occurring as part of the $R^3$ or $R^5$ group can be protected with an ester forming group that will not form a stabilized carbonium ion, for instance the 2,2,2-trichloroethyl group or the *para*-nitrobenzyl group. Such esters are not affected by the process of this invention.

Exemplary of the organic carboxylic acid esters which may be de-esterified by the process of this invention are the diphenylmethyl esters of the amino acids such as glycine, alanine, leucine, threonine, asparagine, glutamine, lysine, arginine, tyrosine, tryptophan, proline, and other amino acids useful in the synthesis of peptides and the like. Typical penicillin esters that may serve as substrates in the present process include the diphenylmethyl, 2,4,6-trimethylbenzyl, 4-methoxybenzyl, and *tert*-butyl esters of penicillin V, penicillin G, penicillin K, and similar penicillins in which $R^3$ in the above formula is methoxybenzylcarbonyl, benzyloxybenzylcarbonyl, methylenedioxybenzylcarbonyl, α-methoxycarbonylbenzylcarbonyl, thienylacetyl, oxazolylacetyl, naphthyloxyacetyl, or benzothiazolyloxyacetyl.

Exemplary of the carbapenams and carbapenems which may be employed as substrates in the present process are the diphenylmethyl esters of thienamycin and related compounds; carbapenams in which X in the above formula is $CH_2$ and $R^3$ is phenylacetyl, phenoxyacetyl, α-aminophenylacetyl α-hydroxyphenylacetyl, furylacetyl, thiazolylacetyl, tetrazolylacetyl, thienylpropionyl, and the like.

Typical cephalosporins which may be employed as substrates include the diphenylmethyl and 4-methoxybenzyl esters of cephalosporin C, cephalexin, cephradine, cefadroxil, cefatrizine, cefaclor, cephalothin, cephapirin, cefacetrile, cephaloridine, cefsulodin, cefazolin, cefamandole, cefuroxime, cefoxitin, cefoperazone, cefotaxime, ceftazidime, ceftezole, and other related cephalosporin esters.

Diphenylmethyl and 2,4,6-trimethylbenzyl esters of carbacephalosporins and 1-oxadethiacephalosporins such as moxalactam also can be used. Typical of such substrates are those of Formula (IV) in which X is $CH_2$ or O and $R^3$ is lower alkanoyl, lower alkoxycarbonyl, cycloalkanecarbonyl, aroyl such as benzoyl, toluoylyl and xyloyl, and groups such as phenylacetyl, phenoxyacetyl, thiazolylacetyl, thienylpropionyl, α-amino-α-phenylacetyl, and other related groups.

It is to be understood that the present ester cleavage process is general and operates equally well on any ester of a carboxylic acid, in which the ester forming group is capable of forming a stabilized carbonium ion. The foregoing list of exemplary substrates is illustrative only and is not intended to be limiting in any respect.

The following non-limiting examples are provided to further illustrate the invention.

Example 1
Removal of 2,4,6-trimethylbenzyl group

A solution of 1.20 g (2.5 mM) of 2,4,6-trimethylbenzyl 7-phenoxyacetamido-3-methyl-3-cephem-4-carboxylate in 17 ml of dichloromethane containing 0.20 ml (2.6 mM) of ethanethiol and 0.34 ml of boron trifluoride diethyl etherate (2.8 mM) was stirred at 25°C for thirty minutes. Thin layer chromatographic analysis showed the presence of 7-phenoxyacetamido-3-methyl-3-cephem-4-carboxylic acid. This compound has the following NMR spectrum: NMR (DMSO-$d_6$): δ 2.10 (s, 3H); δ 3.55 (br s, 2H); δ 4.72 (s, 2H); δ 5.18 (d, 1H); δ 5.73 (dd, 1H); δ 6.87—7.70 (m, 5H); δ 9.30 (d, 1H).

Example 2

Removal of diphenylmethyl group

A solution of 2.0 g (2.8 mM) of diphenylmethyl 7-[α-amino-α-(4-hydroxyphenyl)acetamido]-3-chloro-3-cephem-4-carboxylate paratoluene sulfonic acid salt in 20 ml of dichloromethane containing 0.23 ml (3.1 mM) of ethanethiol and 0.38 ml (3.1 mM) of boron trifluoride diethyl etherate was stirred at 25°C for two hours. The reaction mixture was filtered and the filter cake was washed with fresh dichloromethane and dried for two hours at 40°C in vacuum to provide 1.63 g of 7-[α-amino-α-(4-hydroxyphenyl)acetamido]-3-chloro-3-cephem-4-carboxylic acid.

Example 3

Preparation of 7-[α-amino-α-(4-hydroxyphenyl)acetamido]-3-chloro-3-cephem-4-carboxylic acid

Twenty grams of diphenylmethyl 7-amino-3-chloro-3-cephem-4-carboxylate was acylated by reaction with a mixed anhydride prepared by reacting 4.15 ml of methyl chloroformate with 15.4 g (53.6 mM) of sodium α-N-(methoxycarbonylisopropenyl)amino-α-(4-hydroxyphenyl)acetate to give 40 g of diphenylmethyl 7-[α-N-(methoxycarbonylisopropenyl)amino-α-(4-hydroxypheny)acetamido-3-chloro-3-cephem-4-carboxylate. The latter compound was dissolved in 387 ml of dichloromethane containing 10.20 g (53.6 mM) of *para*-toluenesulfonic acid monohydrate. The reaction mixture was stirred at 25°C for fifteen minutes and then cooled to 10°C. While the reaction mixture was stirred at 10°C there were added 3.89 ml of ethanethiol followed by the dropwise addition over five minutes of 7.69 ml of boron trifluoride diethyl etherate. The reaction mixture was then stirred at 10—15°C for two and one-half hours, whereupon an additional 0.62 ml of boron trifluoride diethyl etherate was added and stirring was continued for an additional ninety minutes. The reaction mixture was filtered and the filter cake was washed with fresh dichloromethane and then dissolved in 100 ml of water and 100 ml of acetone. The aqueous mixture was diluted by addition of triethylamine to pH 4.5, and the crystalline product that formed was collected by filtration and dried to provide 11.98 g of 7-[α-amino-α-(4-hydroxyphenyl)acetamido]-3-chloro-3-cephem-4-carboxylic acid.

NMR(DMSO-$d_6$) δ 3.3—4.1 (m, 2H); 4.8—5.3 (m, 2H); 5.8 (dd, 1H); 6.65—7.2 (two d, 4H); 8.5 (broad, 3H); 9.4 (d, 1H).

Example 4

Removal of 4-methoxybenzyl group

A solution of 2.58 g (5 mM) of 4-methoxybenzyl 7-thiophene-2-acetamido)-3-acetoxymethyl-3-cephem-4-carboxylate in 33 ml of dichloromethane containing 0.39 ml (5.3 mM) of ethanethiol and 0.68 ml (5.5 mM) of boron trifluoride diethyl etherate was stirred at 20°C for two hours and fifteen minutes. Thin layer chromatographic analysis demonstrated complete conversion of the starting material to 7-(thiophene-2-acetamido)-3-acetoxymethyl-3-cephem-4-carboxylic acid.

The reaction mixture was next diluted by addition of 0.83 g (5 mM) of sodium 2-ethylhexanoate, and stirring was continued for ninety minutes. The reaction mixture was added to 25 ml of water and the pH of the mixture was adjusted to 6.4 by addition of saturated aqueous sodium carbonate. The aqueous layer was separated, washed twice with 10 ml portions of dichloromethane, layered with 55 ml of isopropyl acetate and acidified to pH 2 by addition of 20% (v/v) sulfuric acid. The organic layer was separated and dried, and then diluted by addition of 5 ml of methanol containing 0.41 g (5 mM) of sodium acetate. The crystalline product that formed was collected by filtration and air dried to give 640 mg of sodium 7-(thiophene-2-acetamido)-3-acetoxymethyl-3-cephem-4-carboxylate.

NMR(DMSO-$d_6$ + TFA-$d_1$): δ 2.03 (s, 3H); δ 3.8 (br s 2H); δ 4.5—5.25 (m, 3H); δ 5.50—5.59 (m, 1H); δ 6.95 (d, 2H); δ 7.33 (m, 1H).

Example 5

A solution of 4.03 g (5 mM) of diphenylmethyl 7β-[α-*p*-hydroxyphenyl-α-(*p*-methoxybenzyloxycarbonyl)acetamido]-7α-methoxy-3-(1-methyltetrazol-5-yl)thiomethyl-1-oxadethia-3-cephem-4-carboxylate in 33 ml of dichloromethane containing 0.72 ml (10.5 mM) of ethanethiol and 1.36 ml (11 mM) of boron trifluoride diethyl etherate was stirred at 25°C for two and one-half hours. The progress of the reaction was followed by thin layer chromatographic analysis on silica gel coated glass plates, eluting with 1:1:1 (v/v/v) ethyl acetate-toluene-acetic acid. The thin layer chromatograms demonstrated complete removal of both ester groups after about two hours. The reaction mixture was filtered and the filter cake was dried to give 3.43 g of 7β-[α-*p*-hydroxyphenyl-α-carboxyacetamido)-7α-methoxy-3-(1-methyltetrazol-5-yl)thiomethyl-1-oxadethia-3-cephem-4-carboxylic acid. This compound has m.p. 117—122°C (dec.) and IR (KBr): 1780, 1719 and 1632 $cm^{-1}$. The NMR was consistent with that of authentic sample.

By following the general procedures set forth above, ethanethiol and boron trifluoride diethyl etherate is reacted with the following esters to give the corresponding organic acids:

Diphenylmethyl ester of cephalexin to give cephalexin;
2,4,6-Trimethylbenzyl ester of ampicillin to give ampicillin;
*Tert*-butyl ester of cephradine to give cephradine;
Diphenylmethyl ester of thienamycin to give thienamycin;
Pentamethylbenzyl ester of cefadroxil to give cefadroxil;
4-Methoxybenzyl ester of ceftazidime to give ceftazidime;
9-Anthrylmethyl ester of cefoxitin to give cefoxitin.

**Claims**

1. A process for preparing a compound of Formula (I):

$$R^1—COOH \qquad (I)$$

which comprises reacting a lower alkyl thiol and a Lewis acid with an ester of Formula (II):

$$R^1—COOR^2 \qquad (II)$$

in which $R^1$ is the residue of a penicillin, cephalosporin or a nuclear analogue thereof or of an amino acid and $R^2$ is an organic ester forming group capable of forming a stabilized carbonium ion.

2. A process as claimed in Claim 1 in which the lower alkyl thiol is ethanethiol.

3. A process as claimed in Claim 1 or 2 in which the Lewis acid is boron trifluoride.

4. A process as claimed in any one of Claims 1 to 3 in which $R^2$ is diphenylmethyl, 2,4,6-trimethylbenzyl or p-methoxybenzyl.

5. A process as claimed in any one of Claims 1 to 4 in which $R^1$ is a moiety of Formula (IV):

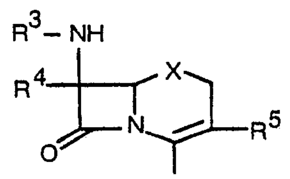

(IV)

in which $R^3$ is an acyl residue, $R^4$ is hydrogen, alkoxy or alkylthio, and $R^5$ is a pharmaceutically acceptable organic group, and X is O, S or $—CH_2—$.

6. A process as claimed in Claim 5 in which $R^5$ is methyl, halo, or acetoxymethyl.

7. A process as claimed in Claim 5 or 6 in which $R^3$ is phenoxyacetyl.

8. A process as claimed in Claim 5 or 6 in which $R^3$ is α-amino-α-(4-hydroxyphenyl)acetyl.

9. A process as claimed in Claim 5 in which $R^3$ is α-carboxy-α-(4-hydroxyphenyl)acetyl, $R^4$ is methoxy and $R^5$ is (1-methyltetrazol-5-yl)thiomethyl.

10. A process as claimed in Claim 5 in which X is $—CH_2—$.

11. A process as claimed in any one of Claims 1 to 4 in which $R^1$ is a moiety of Formula (III):

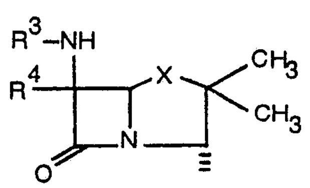

(III)

in which $R^3$ is an acyl residue and $R^4$ is hydrogen, alkoxy or alkylthio, and X is S or $—CH_2—$.

12. A process as claimed in Claim 11 in which X is $—CH_2—$.

13. A process as claimed in any one of Claims 1 to 4 in which a carbapenem ester is used.

14. A process as claimed in Claim 13 in which a thienamycin ester is used.

**Patentansprüche**

1. Verfahren zur Herstellung einer Verbindung der Formel (I)

$$R^1COOH \qquad (I)$$

dadurch gekennzeichnet, daß man ein Niederalkylthiol und eine Lewis-Säure mit einem Ester der Formel (II)

$$R^1COOR^2 \qquad (II)$$

worin $R^1$ der Rest eines Penicillins, Cephalosporins oder eines Kernanalogen hiervon oder einer Aminosäure ist und $R^2$ eine esterbildende organische Gruppe darstellt, die zur Bildung eines stabilisierten Carboniumions befähigt ist, umsetzt.

2. Verfahren nach Anspruch 1, dadurch gekennzeichnet, daß das Niederalkylthiol Ethanthiol ist.

3. Verfahren nach Anspruch 1 oder 2, dadurch gekennzeichnet, daß die Lewis-Säure Bortrifluorid ist.

4. Verfahren nach irgendeinem der Ansprüche 1 bis 3, dadurch gekennzeichnet, daß $R^2$ für Diphenylmethyl, 2,4,6-Trimethylbenzyl oder p-Methoxybenzyl steht.

5. Verfahren nach irgendeinem der Ansprüche 1 bis 4, dadurch gekennzeichnet, daß $R^1$ ein Rest der Formel (IV)

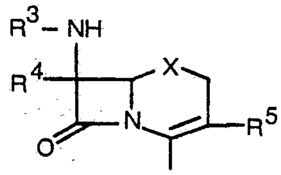

(IV)

ist, worin $R^3$ einen Acylrest bedeutet, $R^4$ für Wasserstoff, Alkoxy oder Alkylthio steht und $R^5$ eine pharmazeutisch annehmbare organische Gruppe darstellt, und X für O, S oder $—CH_2—$ steht.

6. Verfahren nach Anspruch 5, dadurch gekennzeichnet, daß $R^5$ Methyl, Halogen oder Acetoxymethyl ist.

7. Verfahren nach Anspruch 5 oder 6, dadurch gekennzeichnet, daß $R^3$ Phenoxyacetyl ist.

8. Verfahren nach Anspruch 5 oder 6, dadurch gekennzeichnet, daß $R^3$ für α-Amino-α-(4-hydroxyphenyl)acetyl steht.

9. Verfahren nach Anspruch 5, dadurch gekennzeichnet, daß $R^3$ für α-Carboxy-α-(4-hydroxyphenyl)acetyl steht, $R^4$ Methoxy ist und $R^5$ für (1-Methyltetrazol-5-yl)thiomethyl steht.

10. Verfahren nach Anspruch 5, dadurch gekennzeichnet, daß X für $—CH_2—$ steht.

11. Verfahren nach irgendeinem der Ansprüche 1 bis 4, dadurch gekennzeichnet, daß $R^1$ ein Rest der Formel (III)

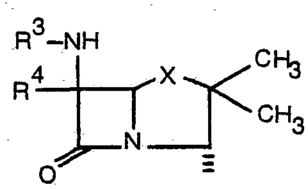

(III)

ist, worin $R^3$ einen Acylrest bedeutet und $R^4$ für Wasserstoff, Alkoxy oder Alkylthio steht und X für S oder $—CH_2—$ steht.

12. Verfahren nach Anspruch 11, dadurch gekennzeichnet, daß X für $—CH_2—$ steht.

13. Verfahren nach irgendeinem der Ansprüche 1 bis 4, dadurch gekennzeichnet, daß ein Carbapenemester verwendet wird.

14. Verfahren nach Anspruch 13, dadurch gekennzeichnet, daß ein Thienamycinester verwendet wird.

**Revendications**

1. Procédé de préparation d'un composé de formule (I):

$$R^1—COOH \qquad (I)$$

caractérisé en ce qu'il consiste à faire réagir un alkyl inférieur-thiol et un acide de Lewis avec un ester de formule (II):

$$R^1—COOR^2 \qquad (II)$$

dans laquelle $R^1$ représente le radical d'une pénicilline, d'une cephalosporine ou d'un de leurs analogues nucléaires, ou d'un amino-acide, tandis que $R^2$ représente un groupe formateur d'ester organique capable de former un ion carbonium stabilisé.

2. Procédé selon la revendication 1, dans lequel l'alkyl inférieur-thiol est l'éthane-thiol.

3. Procédé selon la revendication 1 ou 2, dans lequel l'acide de Lewis est le trifluorure de bore.

4. Procédé selon l'une quelconque des revendications 1 à 3, dans lequel $R^2$ représente le groupe diphénylméthyle, le groupe 2,4,6-triméthylbenzyle ou le groupe p-méthoxybenzyle.

5. Procédé selon l'une quelconque des revendications 1 à 4, dans lequel $R^1$ représente une fraction de formule (IV):

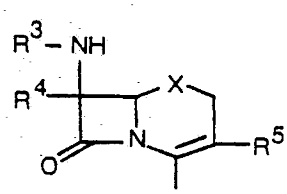

(IV)

dans laquelle $R^3$ représente un radical acyle, $R^4$ représente un atome d'hydrogène, un groupe alcoxy ou un groupe alkylthio, $R^5$ représente un groupe organique pharmaceutiquement acceptable et X représente O, S ou $—CH_2—$.

6. Procédé selon la revendication 5, dans lequel $R^5$ représente le groupe méthyle, un atome d'halogène ou le groupe acétoxyméthyle.

7. Procédé selon la revendication 5 ou 6, dans lequel $R^3$ représente le groupe phénoxyacétyle.

8. Procédé selon la revendication 5 ou 6, dans lequel $R^3$ représente le group α-amino-α-(4-hydroxyphényl)acétyle.

9. Procédé selon la revendication 5, dans lequel $R^3$ représente le group α-carboxy-α-(4-hydroxyphényl)acétyle, $R^4$ représente le groupe méthoxy et $R^5$ représente le groupe (1-méthyltétrazol-5-yl)-thiométhyle.

10. Procédé selon la revendication 5, dans lequel X représente le group $—CH_2—$.

11. Procédé selon l'une quelconque des revendications 1 à 4, dans lequel $R^1$ représente une fraction de formule (III):

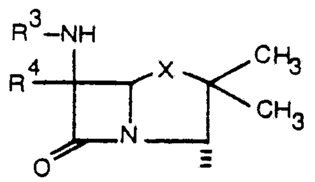

(III)

dans laquelle $R^3$ représente un radical acyle et $R^4$ représente un atome d'hydrogène, un groupe alcoxy ou un groupe alkylthio, tandis que X représente S ou $—CH_2—$.

12. Procédé selon la revendication 11, dans lequel X représente $—CH_2—$.

13. Procédé selon l'une quelconque des revendications 1 à 4, dans lequel on utilise un ester de carbapénem.

14. Procédé selon la revendication 13, dans lequel on utilise un ester de thiénamycine.